(19)

Europäisches
Patentamt
European
Patent Office
Office européen
des brevets

(11)    **EP 2 048 621 B1**

(12)                    **EUROPEAN PATENT SPECIFICATION**

(45) Date of publication and mention
of the grant of the patent:
**26.05.2010 Bulletin 2010/21**

(51) Int Cl.:
**G06T 15/00** (2006.01)          **A61B 6/03** (2006.01)
**G06T 17/00** (2006.01)

(21) Application number: **07118075.6**

(22) Date of filing: **09.10.2007**

(54) **Method and apparatus for volume rendering of medical data sets**

Verfahren und Vorrichtung zur Volumendarstellung medizinischer Datensätze

Procédé et appareil pour rendu de volume d'ensembles de données médicales

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**15.04.2009 Bulletin 2009/16**

(73) Proprietor: **Agfa HealthCare NV
2640 Mortsel (BE)**

(72) Inventors:
• **Kanitsar, Armin Dr.
1100 Wien (AT)**
• **Mroz, Lukas, Dr.
1200 Wien (AT)**
• **Wegenkittl, Rainer, Dr.
3100 Sank Poelten (AT)**
• **Kohlmann, Peter, Dipl.-Inform.
28209 Bremen (DE)**

• **Bruckner, Stefan, Dr.
Vancouver BC, V5N 1V9 (CA)**
• **Gröller, Eduard M., Ao. Univ.-Prof.
1100 Wien (AT)**

(74) Representative: **Linsmeier, Josef
Agfa-Gevaert HealthCare GmbH
Intellectual Property
Tegernseer Landstraße 161
81539 München (DE)**

(56) References cited:
• **KOHLMANN PETER ET AL: "LiveSync: deformed viewing spheres for knowledge-based navigation." IEEE TRANSACTIONS ON VISUALIZATION AND COMPUTER GRAPHICS 2007 NOV-DEC, vol. 13, no. 6, 14 September 2007 (2007-09-14), pages 1544-1551, XP002465946 ISSN: 1077-2626**

## Description

The invention relates to a method and an apparatus for volume rendering of medical data sets according to the preamble of the independent claims.

Modern modalities for medical imaging (e.g. computed tomography) provide large quantities of data at an unprecedented resolution. Presenting this enormous amount of information is a challenging task for today's radiology workstations. Volumetric rendering is the current method of choice for providing a good survey of the data. Combining the information provided by two-dimensional (2D) cross-sections and three-dimensional (3D) visualization can improve the diagnosis process. Linking the different representations of the data has the potential benefit to provide significant enhancements in efficiency. Usually the volumetric display acts as an overview display in this context. The cross-sectional images contain diagnostically relevant information.

Pinpointing a pathological area in the volumetric display selects the corresponding cross-sectional images to be displayed in the two-dimensional display area. From a technical point of view this process is relatively easy to implement. The 3D position of the interesting point can be deduced from the given viewport specification (i.e. transfer function and viewing direction). It is important to note that there is a reduced degree of freedom in highlighting the position on the corresponding cross-sectional image.

The reverse operation is, however, not that straightforward. Picking a two-dimensional position on a cross-sectional slice should result in an expressive unobstructed 3D view. Even though the interesting position is well defined by selecting a point in the cross-sectional image, the appropriate highlighting of the area of interest in the respective 3D rendering is challenging.

The general motivation for emphasizing a structure selected in 2D in its three-dimensional setting is to get the contextual information. A short example illustrates the situation: A frequently occurring request during reading cross-sectional images of computed tomography angiography is to determine to which anatomical structure a specific partially visible vessel belongs. In this case a volumetric rendering of the depicted vessel and its spatial vicinity would be desired. For optimal results the selected structure should be visible to a large extent and must not be occluded by structures of lower importance.

Viewpoint selection is a well investigated research area for polygonal scenes but relatively few research has been done in the scope of volumetric data. Moreover, the combination of optimal viewpoint estimation and synchronized views has received little attention up to now.

Fleishman et al. [6] presented an approach for an automatic placement of the camera for image-based models with known geometry. A quality measure is applied for the visibility and the occlusion of surfaces. Methods like canonical views are investigated by Blanz et al. [2] for aesthetic aspects of a viewpoint. In their experimental setup users assign goodness ratings to viewpoints for three-dimensional object models. Based on the feedback a set of criteria for good viewpoints is defined. To determine the viewpoint quality for virtual scenes Sbert et al. [12] applied a measure based on the Kullback-Leibler distance of the projected area of the polygons in the scene. The mesh saliency approach introduced by Lee et al. [7] measures a regional importance for meshes. Besides mesh simplification this can be employed for viewpoint selection as well. Vázquez et al. [16, 17] worked on the problem that in computer graphics there is no consensus about what defines a good view. Viewpoint entropy based on information theory is introduced to compute good viewing positions automatically. Polonsky et al. [10] aimed for the computation of the best view of an object. They define a set of view descriptors to measure the viewpoint quality. Mühler et al. [8] presented an approach for viewpoint selection in medical surface visualizations. Their work aims at the generation of animations for collaborative intervention planning and surgical education.

Inspired by the research work on polygonal data there is some recent work on viewpoint selection for volumetric data. Bordoloi and Shen [3] presented an entropy-based approach to determine a minimal set of representative views for a given scene. The data distribution, the transfer function and the visibility of voxels are taken into account for their viewpoint selection process. A feature-driven approach to select a good viewpoint is proposed by Takahashi et al. [13]. They identified feature components in the volume for the detection of locally optimal viewpoints. These viewpoints are utilized to extract an optimal global viewpoint. Viola et al. [18] introduced an importance-driven approach to focus on structures within volumetric data. The focus object is defined by the user and their system automatically selects a characteristic viewpoint which provides an expressive view on the object of interest. A framework which facilitates viewpoint selection for angiographic volumes is presented by Chan et al. [5]. View descriptors for visibility, coverage and self-occlusion of the vessels are considered to determine a globally optimal view. This view is selected by a search process in a solution space for the viewpoints.

Besides techniques for viewpoint selection there are numerous approaches to define a region of interest (ROI) in volumetric data. In the scope of volumes this region is also called volume of interest (VOI). Tory and Swindells [14] presented ExoVis for detail and context direct volume rendering. The VOI can be defined by placing a box within the volume. A translation extracts this part from the volume and this 3D cutout can be displayed with different rendering styles or transfer functions. Owada et al. [9] presented volume catcher as a technique to specify a ROI within unsegmented volume data. The user defines this region by drawing a 2D stroke along the contour of the interesting structure and their system performs a constrained segmentation based on statistical region merging. Zhou et al. [20] proposed focal region-guided feature-based volume rendering to emphasize the VOI. In their approach a geometric shape like a sphere is used to divide the volume into a focal and a context region. Regarding tissue classification interesting research has been done by Sato et al. [11]. They have taken 3D local intensity structures into account to identify local features like edges, sheets, lines and blobs which typically correspond to types of tissue in medical volume data. Their local structure filters use gradient vectors along with the Hessian matrix of the volume intensity combined with Gaussian blurring.

In spite of the research it is still complicated and time-consuming to adjust the viewport parameters in order to achieve a good and unobstructed view on the structure of interest. Usually, the user has to take care of the appropriate viewpoint, zooming, transfer function setup, clipping planes and other parameters. This is why in many cases only 2D slices instead of volume renderings of the volumetric data set are examined.

It is an object of the invention to provide a method and an according apparatus enabling good volumetric views of medical data sets and requiring reduced user input.

This object is achieved by the method or the respective apparatus according to the independent claims.

The method comprises the following steps: acquiring a medical data set of an object, in particular a patient, displaying at least one slice view of the acquired medical data set, user-selection of a position on a structure of interest (SOI) in the at least one displayed slice view, displaying a volume rendering of the structure of interest based on one or more first parameters, said first parameters characterizing the view of the displayed volume rendering of the structure of interest, wherein said first parameters are determined automatically by considering the user-selected position and one or more second parameters, said second parameters characterizing at least one of the object, the structure of interest, the currently displayed slice view and one or more previous volume renderings of the structure of interest, and wherein said first parameters are determined in that way that an optimized view on the displayed volume rendering of the structure of interest is achieved without additional user input apart from the user-selection of the position.

The corresponding apparatus comprises a display for displaying at least one slice view of a medical data set of an object, in particular a patient, a user selection unit enabling a user to select a position on a structure of interest (SOI) in the at least one displayed slice view, a volume rendering unit for determining a volume rendering of the structure of interest based on one or more first parameters, said first parameters characterizing the view of the displayed volume rendering of the structure of interest, said volume rendering of the structure of interest being displayed on the display, wherein said volume rendering unit is designed for determining said first parameters automatically by considering the user-selected position and one or more second parameters, said second parameters characterizing at least one of the object, the structure of interest, the currently displayed slice view and one or more previous volume renderings of the structure of interest, and wherein said volume rendering unit is designed for determining said first parameters in that way that an optimized view on the displayed volume rendering of the structure of interest is achieved without additional user input apart from the user-selection of the position.

Relating to the present invention, the one or more first parameters are also referred to as "viewport parameters" and the second parameters are also referred to as "input parameters".

The invention is based on the approach to derive viewport parameters, i.e. first parameters, for a good volumetric view of the selected structure of interest only from the information given by

- the user-selected position on the structure of interest and
- one or more second parameters, wherein the second parameters can be automatically deduced from characteristics of the object, the structure of interest, the currently displayed slice view or one or more previous volume renderings of the structure of interest, respectively.

In this way, good volumetric views of medical data sets are achieved while the necessary user input is reduced to the selection of a position on the structure of interest.

In a preferred embodiment of the invention the one or more first parameters comprise at least one of

- a viewpoint being a point from which the structure of interest is viewed in the displayed volume rendering,
- a viewing direction being a direction in which the structure of interest is viewed in the displayed volume rendering,
- at least one clipping surface, each clipping surface separating the structure of interest into a first and a second region, wherein details of the structure of interest being in the first region of the clipping surface are displayed in the volume rendering whereas details of the structure of interest being in the second region of the clipping surface are not displayed in the volume rendering, wherein at least one clipping surface preferably is a planar clipping plane which is aligned with the viewing direction, and
- a volumetric zoom factor characterizing the size of the structure of interest in the displayed volume rendering.

Hereby, the number of first parameters is reduced to those being most relevant to a good volumetric view so that the deriving of the first parameters is kept simple and fast enabling live synchronized 2D/3D views.

It is also preferred that the one or more second parameters characterizing the object comprise an orientation of the object, in particular a patient orientation, when the medical data set of the object, in particular of the patient, was acquired. Furthermore, it is preferred that the one or more second parameters characterizing the structure of interest comprise information about the shape of the structure of interest. Preferably, the one or more second parameters characterizing the structure of interest comprise information about the visibility of the structure of interest. In another preferred embodiment the one or more second parameters characterizing the one or more previous volume renderings of the structure of interest comprise information about one or more previous viewpoints being the point or points from which the structure of interest was viewed in previously displayed volume rendering or renderings of the structure of interest. Moreover, it is preferred that the one or more second parameters characterizing the currently displayed slice view comprise information about the size of the structure of interest in the currently displayed slice view.

Because the second parameters mentioned above can be easily acquired or deduced automatically from available data; e.g. the medical data set or information regarding the acquisition of the medical data set, no further user input regarding the acquisition or deduction of the second parameters is necessary.

In a preferred embodiment of the invention for at least one of the second parameters an information regarding the quality of viewpoints arising from the at least one of the second parameters is derived. In order to achieve a particularly good view on the picked structure of interest the concept of deformed viewing spheres is used, wherein a viewing sphere surrounds the center of a scanned data set and describes all possible camera positions with respect to this object. The second parameters are utilized to encode the viewpoint quality in deformed viewing spheres whenever a picking action is performed. After combining the deformed spheres for the different second parameters, the estimated quality for all possible viewpoints on the picked structure of interest can be determined from the resulting sphere.

In particular, the information regarding the quality of viewpoints is derived by means of calculating a deformed viewing sphere for at least one second parameter, wherein positions of viewpoints having a higher radial distance from the viewing sphere are regarded to be better than viewpoints having a lower radial distance from the viewing sphere.

In particular, deformed viewing spheres are calculated for two or more second parameters and combined so that a combined deformed viewing sphere is obtained containing information regarding the quality of viewpoints resulting from these second parameters. It is preferred that the deformed viewing spheres for the second parameters are weighted before they are combined to the combined deformed viewing sphere. Particularly, the deformed viewing spheres are combined by summation, multiplication or thresholding of the deformed viewing spheres.

Preferably, a good viewpoint is determined by choosing a viewpoint having substantially the highest radial distance from the viewing sphere of the deformed viewing sphere or of the combined deformed viewing sphere, respectively. It is also preferred that a good viewing direction is defined by the user-selected position and the good viewpoint. In a preferred embodiment, a good clipping surface is positioned by considering the deformed viewing sphere or the combined deformed viewing sphere, respectively, and by considering an accumulated opacity of the structure of interest for rays starting from the user-selected position, wherein the good clipping plane is positioned at a location for which the accumulated opacity is below a given threshold. In another embodiment it is provided that the volumetric zoom factor is determined by considering the size, in particular the slice view zoom factor, of the structure of interest in the currently displayed slice view.

By one or more of the steps mentioned above a simple and quick deriving of the first parameters is achieved.

An advantageous embodiment of the invention regarding a live synchronization of 2D/3D views is characterized in that several positions are user-selected by successively pointing to different positions on the structure of interest in the displayed slice view and wherein for each position of the several positions said first parameters are determined automatically and the display of the corresponding volume rendering of the structure of interest is updated successively in that way that an optimized view on the displayed volume renderings of the structure of interest is achieved without additional user input apart from successively pointing to the different positions on the structure of interest.

It is particularly preferred that a plurality of positions on the structure of interest are user-selected by continuously tracing along the structure of interest in the displayed slice view and wherein for each position of the plurality of positions said first parameters are determined automatically and the display of the corresponding volume rendering of the structure of interest is updated continuously in that way that an optimized view on the displayed volume renderings of the structure of interest is achieved without additional user input apart from continuously tracing along the structure of interest. This embodiment is a very advantageous application of the live synchronization of 2D/3D views according to the invention.

Additionally or alternatively, one or more positions on the structure of interest are selected automatically, i.e. without user interaction. Preferably, the structure of interest and its shape and/or run are identified automatically and the positions on the structure of interest are positioned automatically along the identified shape and/or run of the structure of interest. E.g. a cardiovascular data set is analyzed and the vessels are identified and described via a centre line running along the centre of the vessels. Hereupon, one or more positions along said centre line are selected automatically and the volumetric view is set up or updated accordingly. Thus, no user interaction is necessary for achieving a good volumetric view on the relevant vessels of the cardiovascular data set.

It is also preferred that the automatic determination of said first parameters and the subsequently updated display of the corresponding volume renderings can be activated and deactivated by the user. In this embodiment, activation and deactivation can happen by pressing and releasing an activation key, in particular a function key or control key, or by selecting an icon on a display. In particular, the automatic determination of said first parameters and the subsequently updated display of respective volume renderings take place only when the position on the structure of interest in the displayed slice view is selected by the user while the activation key is simultaneously pressed by the user. In a further embodiment of the invention the user deactivates the automatic determination of said first parameters and the subsequent display of respective volume renderings and amends at least one of the automatically determined first parameters, whereupon an updated volume rendering of the structure of interest is displayed based on the amended first parameters.

In a further or alternative embodiment of the invention at least two deformed viewing spheres are obtained from previously acquired medical data sets of one or more objects, in particular one or more patients, both said previously acquired medical data sets and a currently acquired medical data set originating from the same type of examination, wherein first parameters characterizing the view of the displayed volume rendering of a structure of interest of the currently acquired medical data set are determined by considering the at least two deformed viewing spheres obtained from the previously acquired medical data sets.

This embodiment is based on the approach to derive at least one first parameter, in particular a viewpoint, characterizing the volume rendering of the currently acquired medical data of a patient from deformed viewing spheres that were calculated from previously acquired medical data of said patient and/or another patient and/or other patients. By utilizing previously obtained information in this way a good volumetric view on the currently acquired medical data set is obtained readily.

Preferably, the first parameters derived in this way are used for an initial display of the volume rendering of the currently acquired medical data. Subsequently, a determination of the first parameters based on one or more second parameters characterizing at least one of the currently examined patient, the currently selected SOI, the currently displayed slice view and already displayed volume renderings of the currently selected SOI can be effected as described in detail above.

It is preferred that said at least two deformed viewing spheres obtained from the previously acquired medical data sets are superimposed so that an accumulated deformed viewing sphere is obtained and that the first parameters are derived from said accumulated deformed viewing sphere. In this preferred embodiment, the information contained in different deformed viewing spheres calculated from medical data of the same or different patients but originating from the same medical examination type, e.g. computed tomography images of patients' heads, is accumulated or superimposed, e.g. by adding and/or averaging, in the accumulated deformed viewing sphere. From said accumulated deformed viewing sphere first parameters resulting in a good initial view of the currently acquired medical data of the currently examined patient can be deduced easily and very quickly.

It is also preferred that at least one of said at least two deformed viewing spheres obtained from the previously acquired medical data sets can be a combined deformed viewing sphere that is obtained by combining two or more viewing spheres for two or more second parameters characterizing at least one of the previously examined patient, previously selected SOI, previously displayed slice view and previous volume renderings of the previously selected SOI. The combination of deformed viewing spheres to a combined deformed viewing sphere and its advantages were already described above.

In summary, the invention provides a new concept to synchronize 2D slice views and 3D views of medical data sets. Through intuitive picking of a position on a displayed 2D slice view, the user defines the anatomical structures he or she is interested in. The 3D volumetric view is updated automatically with the goal that the user is provided with expressive 3D images. To achieve this real-time or "live" synchronization - hence the term "LiveSync" for the invention - a minimal set of second parameters is used without the need for segmented data sets and data-specific pre-computations etc. The only necessary user interaction to derive all the viewport parameters, i.e. the first parameters, based on the second parameters is given by the picking of a position on a displayed slice view.

In the following, the invention is described in more detail referring to the figures, wherein

Fig. 1 shows a preferred workflow according to the invention;

Fig. 2 illustrates the patient orientation viewing sphere;

Fig. 3 shows examples for the first principal component determined by PCA;

Fig. 4 illustrates the viewpoint history viewing sphere;

Fig. 5 illustrates local shape estimation viewing spheres;

Fig. 6    illustrates visibility viewing spheres;

Fig. 7    illustrates the effect of different operators in the combination of viewing spheres;

Fig. 8    shows a first application example of the invention;

Fig. 9    shows a second application example of the invention;

Fig. 10   shows a third application example of the invention; and

Fig. 11   shows an example of an apparatus for volume rendering of medical data sets according to the invention.

<u>APPARATUS AND WORKFLOW</u>

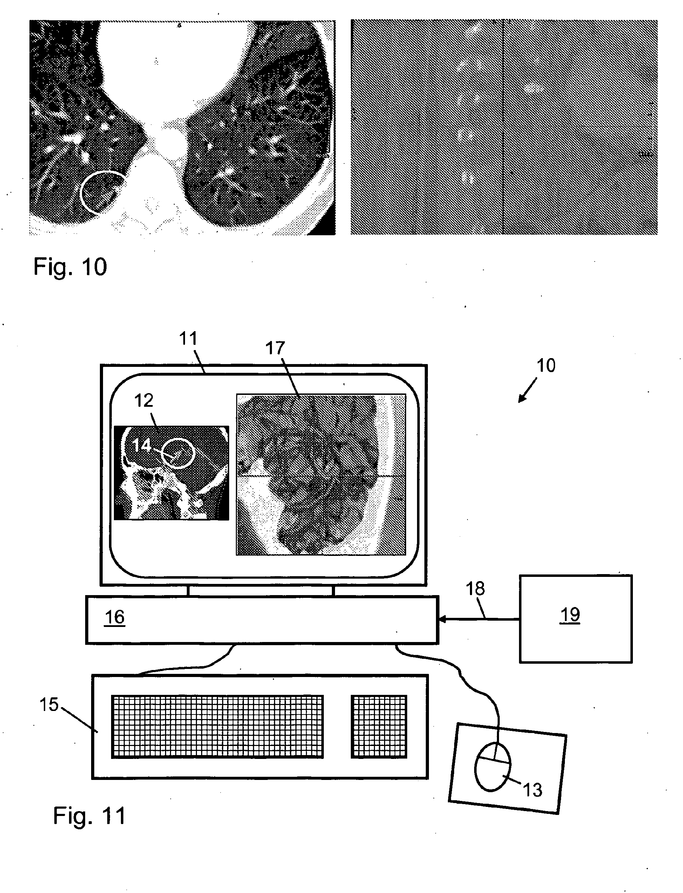

Fig. 11 shows a schematic representation of an example of an apparatus 10 for volume rendering of medical data sets according to the invention. A medical data set 18 is generated by a medical imaging system 19, e.g. an x-ray or CT apparatus, and is fed to the apparatus 10.

The apparatus 10 comprises a display 11, e.g. a TFT screen, for displaying a slice view 12 of a medical data set of an object, which is a patient in this case, and a mouse 13 serving as a user selection unit enabling the user to select a position on a structure of interest (SOI) in the displayed slice view 11 by moving a pointer 14 to the structure of interest on the displayed slice view 12 and by pressing and/or releasing a key on the mouse 13 or on the keyboard 15, e.g. a control key or hot key. The apparatus 10 further comprises a volume rendering unit 16 for determining a volume rendering 17 of the selected structure of interest being displayed on the display 11.

To enable a 2D/3D synchronization of the displayed slice view 12 and the volumetric view 17, the functionality of LiveSync can preferably be activated by pressing a hot key on the keyboard 15 while pointing with the mouse pointer 14 on the structure of interest on the slice 12 and can be deactivated by releasing the hot key.

Based on this picking process, knowledge-based techniques are applied to estimate good viewpoints for the volumetric view, to calculate an appropriate placement of a view-aligned clipping plane, and to adjust the zoom factor.

Depending on the user's preferences, the apparatus 10 allows a smoothly animated rotation or an instant switch between two successive viewpoints. In the case the user is not entirely satisfied with a provided volumetric view 17, it can be refined by manually changing the viewpoint, replacing the clipping-plane, or adjusting the proposed zooming to get a better view of the SOI.

If LiveSync is not activated the navigation with the slices is done in a traditional manner and does not lead to an update of the volumetric view.

The following factors are considered to achieve the live synchronization according to the invention:

- Picked point (i.e. user-selected position): The volumetric position of the depicted structure is determined by the position which the user has picked a position on a slice 12.
- Slice view zoom: The zoom of the slice view 12 serves as an indicator for the size of the interesting anatomical structure. To set up all first parameters automatically this zoom factor is considered to adjust the zoom of the volumetric view 17.
- Patient orientation: Scanned medical data contain information about the patient's position and orientation when the medical data were acquired. Taking into account knowledge about the performed procedure, a rough estimate of the preferred viewing directions is possible.
- Viewpoint history: The last viewpoint is used as a parameter for the selection of the next viewpoint. This means that the system tries to find a good viewpoint close to the last one if this does not counteract the other parameters.
- Local shape estimation: The local shape of the picked structure is estimated based on local segmentation. Three major shapes - lines, sheets and blobs - are assigned to structures to be utilized as parameters for viewpoint selection.
- Visibility: Another parameter is the visibility of the picked structure. To compute visibility, rays are cast from the picked position to a certain number of viewpoints and analyzed regarding occluding structures.

The second parameters patient orientation, viewpoint history, local shape estimation and visibility are encoded directly in the viewing spheres. If the particular parameter indicates a good viewpoint at a certain position, a unit sphere is deformed in a way that the distance of this point to the sphere's center is increased.

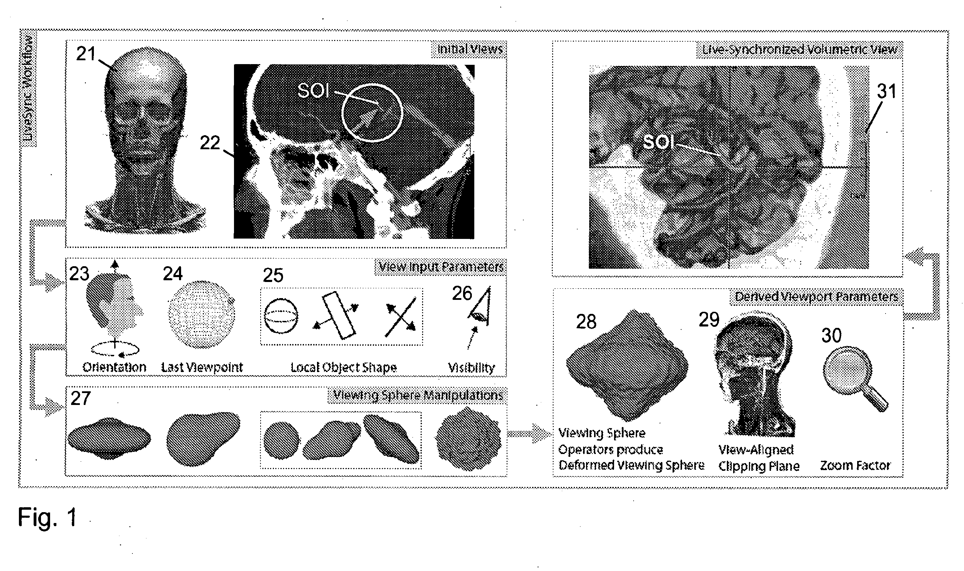

Fig. 1 gives an overview on the LiveSync workflow. Initially there is a volumetric view 21 which is shown from a default viewpoint and a 2D slice image 22. Each picking action on the displayed slice 22 initiates a deformation of viewing spheres for at least one of the following second parameters: patient orientation 23, viewpoint history 24, local shape estimation 25, and visibility 26. In this overview, the second parameters are referred to as "view input parameters".

The corresponding deformed viewing spheres 27 of these second parameters are weighted and combined to get a resulting combined deformed sphere 28 which encodes the combined quality of the viewpoints.

In addition, the zoom factor 30 is adjusted and a view-aligned clipping plane 29 is positioned allowing a flexible removal of occluding structures to generate a meaningful volumetric view 31.

In this way, for each picking action on the slice 22, the second parameters 23 to 26 are used to estimate good viewpoints and to deform the viewing spheres 27 accordingly so that a live synchronized volumetric view 31 is generated automatically providing a good view on the picked structure without any further user input, data-specific a priori information or pre-computations.

DEFORMED VIEWING SPHERES

The concept of viewing spheres is used for setting up the viewpoint and the viewing direction. Basically, a virtual camera can be placed at any point on the surface of a sphere which encapsulates the scene. To move the camera on this sphere typically rotation operations are performed. In addition, the viewing direction of the camera defines on which location in the scene the camera is focusing. Zooming can be achieved by moving the camera along the surface normal of its position on the sphere.

Sphere Parameterization

As the second parameters have to be encoded directly into the sphere's shape there is need for an intuitive way to parameterize the viewing sphere. In addition, this parameterization has to be stored efficiently with taking into consideration that operators for the combination of the individual spheres have to be applicable. A convenient parameterization of spheres can be achieved with polar coordinates. In this system each point of a sphere can be characterized by $\Theta$ and $\varphi$, which represent the polar and the azimuthal angle, and its radial distance r. The polar angle starts from the positive z-axis and ranges from 0 to 180° and the azimuthal angle in the xy-plane starts from the positive x-axis with a range from 0 to 360°. With this parameterization several conversions and calculations can be computed very efficiently [15, 19].

Sphere Map

A well-known challenge in computer graphics is the problem of applying a texture map to a sphere. The naive approach performs a direct latitude-longitude mapping onto a sphere by using a single rectangular texture in which the width is twice the height. With the so-called uv-mapping *u* spans the equator and *v* covers the pole-to-pole range. This is a straightforward mapping with the disadvantage that the sampling becomes higher towards the pole regions. Alternatives for spherical textures are cube, omnitect, icosahedral and octahedral mappings [1].

The inverse problem has to be handled to map a sphere to a structure which facilitates the operations that are performed in the present invention. Because of memory efficiency and intuitive indexing a direct latitude-longitude mapping is preferred, wherein the rectilinear texture is stored as a two-dimensional array with $360 \times 180$ entries. Explicit storing in memory is necessary to facilitate an efficient combination of differently sampled data. In the current implementation information about patient orientation, viewpoint history and local shape estimation is analytically described, whereas visibility information is sampled in a discrete manner. As the angular position can be calculated from the array indices it is sufficient to write the radial distance values to this array.

Sphere Deformation

The general idea to indicate the quality of viewpoints is the direct deformation of the viewing sphere. Positions on the sphere's surface with a high radial distance represent good viewpoints. To achieve an appropriate deformation of the sphere, the Phong illumination model serves as an analogy. In this model a hemisphere represents the diffuse reflection intensity with a bump which indicates the specular reflection intensity. Phong's model of the specular highlight is adapted for the calculation of the radius *r* at a certain point on the sphere's surface with the following Equation 1:

$$r = a \cdot (\boldsymbol{n} \bullet \boldsymbol{v})^{mw}, \qquad (1)$$

where a is a constant which controls the height of the bump, *n* is the surface normal at a specific point on the sphere, *v* is the surface normal at a good viewpoint and *mw* controls the width of the bump. With slight variations of this formula the deformed spheres for most second parameters used for viewpoint selection can be generated.

VIEWING SPHERE MANIPULATORS

A challenging part in the selection process of a good viewpoint is the identification of the relevant parameters. For a generic solution which works for different types of medical volume data, the definition of the second parameters is important. It was found that the patient's orientation, the viewpoint history, the local shape of the structure and its visibility are of high relevance for viewpoint selection. The viewing spheres are deformed to encode the viewpoint quality for each of these second parameters.

Patient orientation viewing sphere

The first utilized second parameter to construct a deformed viewing sphere is the patient's orientation. According to the type of an examination there exist general preferred viewing directions. In this case the head-feet axis serves as a rough estimation to derive the preferred viewpoints.

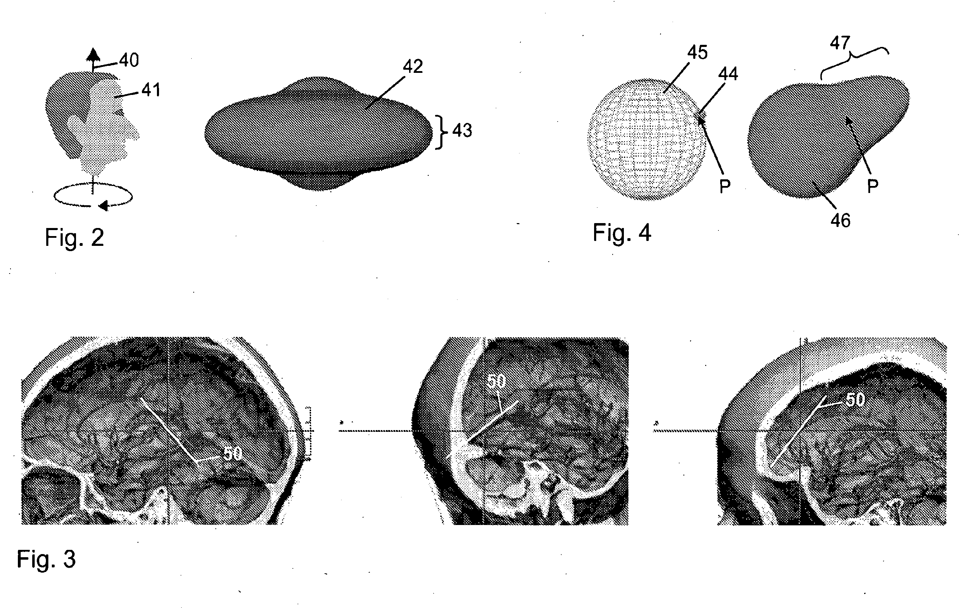

Fig. 2 (left) shows the rotation axis 40 which corresponds to the patient's 41 orientation. The corresponding viewing sphere 42 as shown in Fig. 2 (right) is deformed in a way that it prefers viewpoints which are orthogonal to this axis 40, i.e. it is enlarged around the equator 43. This deformation is achieved by applying Equation 1 as described in the following Algorithm 1 where the z-axis is the main rotation axis 40:

------------------------------------------------------------------------------

for each line of longitude *lon* do

    set *v* to the surface normal at a latitude of 90°

    for each parametrized point *p* of *lon* do

        set *n* to the surface normal at *p*

        compute radius at this point with Equation 1

    end for

end for

------------------------------------------------------------------------------

Viewpoint history viewing sphere

The selection of a good viewpoint is based on different second parameters to provide the user with an intended view. As a specific view was selected by the system based on estimated demands of the user, the current viewpoint will also be considered for the estimation of the quality of the next viewpoints. Especially, big shifts of the viewpoint for two successive pickings should be avoided if possible. This means that if there is a good viewpoint for the picked structure close to the current one this viewpoint is preferred to others which are positioned farther away on the viewing sphere.

Fig. 4 shows how a deformed viewing sphere 46 for this criterion looks like. The position P of the last viewpoint 44 is marked on the viewing sphere 45 (left). After deformation the resulting deformed viewing sphere 46 has a bump 47 with a maximum at this position P which also encodes the quality of surrounding viewpoints. The according deformation can be generated with the following Algorithm 2:

---------------------------------------------------------------------------

```
set v to the surface normal of the last viewpoint

for each point p of the parameterized sphere do

        set n to the surface normal at p

        if dot(v,n)>0 then

                compute radius at p with Equation 1

        else

                set radius to 1

        end if

end for
```

---------------------------------------------------------------------------

Local shape estimation viewing-sphere,

Another important second parameter for viewpoint selection is the local shape of the structure of interest (SOI). If the picked point is e.g. part of a blood vessel, a good viewpoint shows the course of this vessel and does not cut through it. With a fast local segmentation and a principal component analysis (PCA) the shape information can be derived locally from the data values. Region growing is performed on a $32\times32\times32$ neighborhood of the picked data point which serves as seed point. The lower and upper threshold for the region growing are calculated by analyzing the distribution of the scalar values at the picked point and its neighborhood. The result of this local segmentation is a connected 3D point cloud. PCA is performed on this point cloud to extract the three feature vectors and the corresponding eigenvalues which are utilized to determine the local feature shape according to a metric of Sato et al. [11].

Fig. 3 shows how the vector of the first principal component is oriented when picking is performed at three different positions on blood vessels in the head. The white line 50 displays the respective orientation of the most important feature vector determined by a PCA for three different positions on blood vessels in the head. These vectors are strongly aligned with the local orientation of the vessels. The local orientation of the vessels is indicated by these vectors quite well.

In combination with the orthogonal second and third principal components and the corresponding eigenvalues this information is used to create the deformed spheres for the local shape estimation.

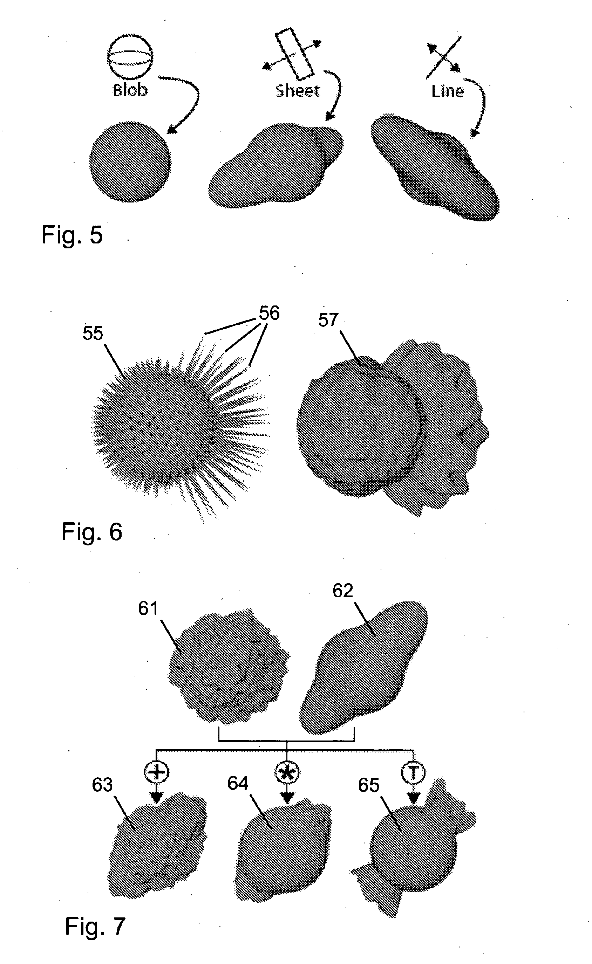

According to the local shape of the object, the viewing sphere has to be deformed as illustrated in Fig. 5. If the object has a volumetric extent (see "Blob"), then basically all viewpoints are of the same quality (left). For a planar structure (see "Sheet") the viewpoints which are orthogonal to the sheet are favored (middle). If a tubular structure (see "Line") is determined, the preferred viewpoints are aligned along a ring which is orthogonal to this line (right).

For the planar object the deformation of the sphere is calculated in analogy to the deformed sphere for the viewpoint history (see above). To get two bumps on the opposite sides of the sphere Equation 1 is adjusted slightly to Equation 2:

$$r = a \cdot abs((\boldsymbol{n}\bullet\boldsymbol{v})^{mw}). \quad (2)$$

If the structure is tubular the deformation process is a bit more complex. It is a generalization of the deformation process of the patient orientation viewing sphere because the tube can be oriented arbitrarily within the volume. Geometrically the good viewpoints are located around a great circle of the viewing sphere, defined by the two points where the vectors of the second and the third principle components intersect the sphere's surface. A great circle is always uniquely defined by two points on the surface of the sphere, and its center is the same as the center of the sphere. For each position $p$ on the sphere's surface the vector from the origin to the closest point on the great circle has to be calculated. This can be achieved by projecting the vector from the origin to $p$ onto the plane of the great circle. The procedure to generate the deformed sphere is presented in following Algorithm 3:

```
if shape == blob then
        radius of each point of the parameterized sphere is 2
else if shape == sheet then
        set v to the vector of the third principal component
        for each point p of the parameterized sphere do
                set n to the surface normal at p
                compute radius at p with Equation 2
        end for
else if shape == line then
        calculate great circle c for the two points where the 2nd and the 3rd
        principal component intersect the surface of the unit sphere
        for each point p of the parameterized sphere do
                set n to vector from the origin to p
                set v to the projection of n onto the plane of c
                normalize v
                compute radius at p with Equation 1
        end for
end if
```

Visibility viewing sphere

A further building block for estimating a good viewpoint is defined by the visibility information. Starting from the picked point visibility rays are cast to determine occluding objects. As mentioned above, the parameterized points of the sphere are not distributed uniformly. It is neither efficient nor necessary to cast visibility rays to all 360 × 180 positions. Nevertheless, it is highly preferable that the positions which are tested are distributed uniformly on the sphere. Bourke [4] provides source code (written by Lettvin) for this purpose. Based on the standard physics formula for charge repulsion an arbitrary number of points is distributed over the surface of a sphere. It was found that a subset of 36 × 18 rays provides a good trade-off between performance and quality. The calculation of the uniformly distributed points is performed only once and the result is stored in a look-up table.

To determine whether a certain viewpoint provides good visibility of the selected structure, rays are cast from the picked point. As a local segmentation was performed for the local shape estimation, this information is utilized to determine when a ray exits the tissue of interest. When this has happened the opacity information of the transfer function is considered. The opacity is accumulated along the ray and as soon as a small opacity threshold is surpassed the calculation is terminated for the specific ray. A high visibility value is assigned to a viewpoint if there is much space from the picked point in the direction of this viewpoint until it gets occluded by other structures. Such a situation provides more flexibility for positioning the clipping plane. This allows to position the clipping plane orthogonal to the viewing direction far away from the picked point, so that an unobstructed view of the picked point is possible while the helpful context information is not unnecessarily reduced.

The corresponding deformed viewing sphere 55 is depicted in Fig. 6. The lengths of the spikes 56 encode the viewpoint quality at a uniformly distributed set of sample positions (left). After reconstructing at all positions a smooth sphere 57 is generated (right).

One important criterion for the viewpoint entropy of Bordoloi and Shen [3] is view stability which describes the maximum change in a certain view caused by small camera shifts. The view is defined to be stable if a small camera change implies also only small changes in the view. Transferred to the visibility viewing sphere there is the possibility to encode view stability derived from the visibility values at the discrete uniformly distributed points. It is heuristically assumed that a viewpoint in between several good ones is also rather good. Such a point offers high view stability, because small changes of the viewpoint will also lead to good viewpoints. To encode this information into the viewing sphere, for all the parameterized sphere positions which are not explicitly tested for visibility a weighting with the surrounding tested points is performed. With this weighting a smoothly deformed sphere 57 is obtained as shown in Fig. 6 (right). The pseudo code to generate the deformed sphere 55 for the visibility criterion is presented in following Algorithm 4:

```
_____

for each of the uniformly distributed points p do

        calculate the visibility

        set the radius at p to the visibility value

end for

for each point s of the parameterized sphere do

        get all p's within a certain distance d to s

        for each p in range d do

                set n to the surface normal at p

                set v to the surface normal at s

                compute r with Equation 1

                add r to the current radius at s

        end for

        normalize the radius at s

end for

_____
```

VIEWING-SPHERE OPERATORS

After the generation of the deformed viewing spheres for the various second parameters they are weighted and combined in order to factor in all the effects.

Weighting of viewing spheres

Equation 1 offers different options to weight the extent of deformation of a sphere. Basically, *a* controls the height of the bump and *mw* its width. To facilitate the combination operators the values of a for the individual sphere deformations are chosen so that their radii vary from 1 to 2 after deformation. For all the second parameters it was found that an estimated good viewpoint also influences the quality of viewpoints in a certain neighborhood. For each viewpoint criterion the radius can vary by a factor of two around a good viewpoint at a certain position. The sphere generation for the viewpoint history contains a built-in weighting control. A big shift of the viewpoint is quite disturbing for picking actions within a small spatial area of the data but it is acceptable for two picked points which are located far apart from each other. This just means that the user is switching to a totally different inspection region whereby the viewpoint coherency is less critical.

To take this into account, a distance factor *d* is calculated being the ratio of the spatial distance between two successively picked points to the diagonal extent of the volume. To influence the weighting for the viewpoint history viewing sphere Equation 1 is modified to

$$r = (1-d) \cdot a \cdot (\mathbf{n} \bullet \mathbf{v})^{mw}. \quad (3)$$

Combination of viewing spheres

As the deformed spheres were calculated for the second parameters individually, they have to be combined into a single sphere, i.e. a combined deformed viewing sphere, which encodes the overall viewpoint quality. Preferably, three operators are implemented for this combination, namely summation, multiplication and thresholding. Each of these operators emphasizes certain viewpoint characteristics.

Fig. 7 shows the effects of the three operators on the resulting sphere. For this example a visibility viewing sphere 61 and a local shape estimation viewing sphere 62 are chosen as input spheres. The application of the operators and the development of additional operators is easy to achieve because each deformed sphere 61, 62 is parameterized as a two-dimensional array.

As operands for the operators, the offset of the radius which is higher than the radius of the unit sphere is taken. At each position the radius of a deformed sphere 61, 62 has a value between 1 and 2 so that the operations are performed on values between 0 and 1. The implementation and the characteristics of the realized operators are as follows:

- Summation (+): A loop over all entries of the sphere arrays is performed to sum up the corresponding radii. This intuitive approach leads to very good results. Good viewpoints will be detected at positions of the combined deformed viewing sphere 63 where at least some of the input spheres 61, 62 indicate a good one. Summation is not as sensitive to outliers as multiplication or thresholding.
- Multiplication (*): To emphasize certain characteristics more strongly an operator is implemented which computes the multiplication of the input spheres 61, 62. This operator emphasizes positions where good viewpoints are indicated by several source spheres (cf. deformed viewing sphere 62) and deemphasizes positions where at least one source sphere (cf. deformed viewing sphere 61) indicates a bad viewpoint. Low values have an increased impact on the result. Even if the value of only one input sphere is low the corresponding viewpoint on the combined deformed viewing sphere 64 will be rated as a bad one.
- Thresholding (T): For the thresholding operation one specific sphere is taken as the initial one. In a loop over all parameterized points of this sphere the value at this position is only considered if the values of the other spheres at the same position are above a certain threshold. If this is not the case the radius on the specific position is set to 1. This operator filters out the values where the corresponding values on the other spheres are indicating a bad viewpoint. With the thresholding it is possible to define knockout criteria. Assuming the patient orientation viewing sphere (see 42 in Fig. 2) being the initial thresholding sphere a window for a certain preferred viewing direction can be defined. By thresholding over the other deformed viewing spheres a good viewpoint within this frame will be estimated.

DERIVING OF FIRST PARAMETERS

After describing the second parameters, the viewing sphere manipulators and the viewing-sphere operators, the deriving of the first parameters for setting up the volumetric view will be explained in the following. First parameters are a good viewpoint, the placement of the view-aligned clipping plane, the zoom and the viewing direction.

The application of the viewing sphere operators to the individual deformed viewing spheres produces a combined viewpoint quality map at 360 × 180 positions on the combined deformed viewing sphere. Thus, a good viewpoint can be easily determined by the highest entry in the combined deformed viewing sphere map array which holds the radial distances of all points. The volumetric data can then be displayed on the display 11 of the apparatus 10 (see Fig. 11) according to the best estimated viewpoint or suggest a small number of preferred views (e.g., displayed as thumbnails).

With the information obtained by the visibility calculation (cf. section "Visibility viewing sphere"), the exact position where the picked point is occluded along each tested visibility ray is known. This information is used for setting up of a view-aligned clipping plane to clip away the occluding structures. To position the clipping plane, a location along the ray starting at the picked point where the accumulated opacity is still below a small threshold is selected. This allows an unobstructed view of the picked object while preserving as much context information as possible.

The viewing direction is directly defined by the picked point and this point is shown in the center of the volumetric view window 17 (see Fig. 11).

Finally, the zoom factor for the volumetric view 17 can be derived from the current settings of the displayed slice view 12. The zoom of the slice view 12 gives a rough estimation about the size of the interesting anatomical structure. In the current implementation this zoom factor directly determines the zoom of the volumetric view 17.

APPLICATION EXAMPLES

Preferably, the invention is executed on a medical computer workstation. The corresponding computations for the LiveSync viewpoint selection can be performed interactively and are not influenced very much by the size of the medical data set. E.g., on a PC configured with an AMD Athlon 64 Dual Core Processor 4400+ and 2 GB of main memory the LiveSync-related computations take about 70 ms to 150 ms per picking, depending on the number of segmented voxels at the local segmentation step and on the estimated local feature shape. Thus, the user gets an almost instant update of the volumetric view whenever he or she is picking on a certain structure in a 2D slice. To demonstrate the usefulness of the interactively synchronized views the results for three different application scenarios will be discussed in the following.

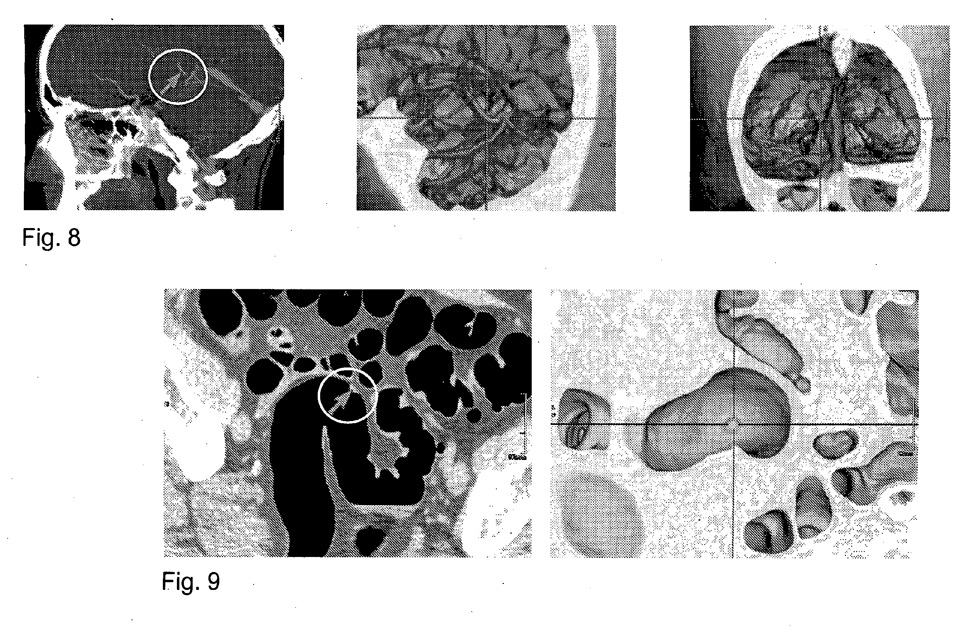

In a first example an estimated good viewpoint and a rather bad viewpoint are presented for comparison reasons. Fig. 8 shows the results for a picking action on a partly visible vessel in a 2D slice image (left). The corresponding volumetric view (middle) is based on a viewpoint which was determined by the method according to the invention and is very good. Obviously, the information about the vessel's course and its spatial vicinity can be recognized very easily.

In contrast to this, a viewpoint which is rated to be rather bad leads to a volumetric view as shown in Fig. 8 (right). Important parts of the vessel are occluded, its course remains unclear and the connectivity to other vessels can hardly be revealed by this viewpoint.

The following two application scenarios demonstrate that LiveSync is a generic tool for various kinds of clinical examinations. In their typical workflow radiologists search for specific structures in medical data. Although there exist highly sophisticated and specialized methods, e.g. for the detection of polyps in the colon or lung nodules, LiveSync can help to quickly explore theses pathological cases.

The examination of the colon is a very difficult task with 2D slices only because it is very hard to see differences between foldings of the colon and polyps. Fig. 9 shows the LiveSync result (right) for the picking (left) of a suspicious structure in the colon. With the provided volumetric view it can be clearly seen that the picked structure is not a colon folding but a polyp.

Another challenging task is the detection of lung nodules. In the 2D slices they often look very similar to bronchia or vessels. In Fig. 10, a structure which is assumed to be a nodule is picked on the slice and LiveSync presents the corresponding volumetric view automatically. This view can clearly help to classify the picked structure as a lung nodule.

REFERENCES

[1] Virtual Terrain Project. Available online at http://www.vterrain.org/Textures/spherical.html, March 2007.

[2] V. Blanz, M. J. Tarr, and H. H. Bülthoff. What object attributes determine canonical views? Perception, 28: 575-599, 1999.

[3] U. D. Bordoloi and H.-W. Shen. View selection for volume rendering. In IEEE Visualization '05, pages 487-494, 2005.

[4] P. Bourke. Distributing Points on a Sphere. Available online at http://local.wasp.uwa.edu.au/~pbourke/geometry/spherepoints/, March 2007.

[5] M.-Y. Chan, H. Qu, Y. Wu, and H. Zhou. Viewpoint selection for angiographic volume. In International Symposium on Visual Computing '06, pages 528-537, 2006.

[6] S. Fleishman, D. Cohen-Or, and D. Lischinski. Automatic camera placement for image-based modeling. Computer Graphics Forum, 19(2):101-110, 2000.

[7] C. H. Lee, A. Varshney, and D. W. Jacobs. Mesh saliency. In ACM SIGGRAPH '05, pages 659-666, 2005.

[8] K. Mühler, M. Neugebauer, C. Tietjen, and B. Preim. Viewpoint selection for intervention planning. In Eurographics/IEEE VGTC Symposium on Visualization, pages 267-274, 2007.

[9] S. Owada, F. Nielsen, and T. Igarashi. Volume catcher. In Symposium on Interactive 3D Graphics and Games '05, pages 111-116, 2005.

[10] O. Polonsky, G. Patané, S. Biasotti, C. Gotsman, and M. Spagnuolo. What's in an image: Towards the computation of the "best" view of an object. The Visual Computer, 21(8-10):840-847, 2005.

[11] Y. Sato, C.-F. Westin, A. Bhalerao, S. Nakajima, N. Shiraga, S. Tamura, and R. Kikinis. Tissue classification based on 3D local intensity structures for volume rendering. IEEE Transactions on Visualization and Computer Graphics, 6(2):160-180, 2000.

[12] M. Sbert, D. Plemenos, M. Feixas, and F. González. Viewpoint quality: Measures and applications. In Computational Aesthetics '05, pages 185- 192, 2005.

[13] S. Takahashi, I. Fujishiro, Y. Takeshima, and T. Nishita. A feature-driven approach to locating optimal viewpoints for volume visualization. In IEEE Visualization '05, pages 495-502, 2005.

[14] M. Tory and C. Swindells. Comparing ExoVis, orientation icon, and inplace 3D visualization techniques. In Graphics Interface '03, pages 57-64, 2003.

[15] J. M. van Verth and L. M. Bishop. Essential Mathematics for Games and Interactive Applications. Morgan Kaufmann Publishers, San Francisco, California, 2004.

[16] P.-P. Vazquez,M. Feixas,M. Sbert, and W. Heidrich. Viewpoint selection using viewpoint entropy. In VMV '01, pages 273-280, 2001.

[17] P.-P. Vázquez, M. Feixas, M. Sbert, andW. Heidrich. Automatic view selection using viewpoint entropy and its application to image-based modelling. Computer Graphics Forum, 22(4):689-700, 2003.

[18] I. Viola, M. Feixas, M. Sbert, and M. E. Gröller. Importance-driven focus of attention. IEEE Transactions on Visualization and Computer Graphics, 12(5):933-940, 2006.

[19] E. Williams. Aviation Formulary V1.43. Available online at hftp://williams.best.vwh.net/avform.html, March 2007.

[20] J. Zhou, M. Hinz, and K. D. Tönnies. Focal region-guided feature-based volume rendering. In 1st International Symposium on 3D Data Processing, Visualization and Transmission '02, pages 87-90, 2002.

**Claims**

1. Method for volume rendering of medical data sets comprising the following steps:

   - acquiring a medical data set of an object, in particular a patient,
   - displaying at least one slice view (12, 22) of the acquired medical data set,
   - user-selection of a position on a structure of interest (SOI) in the at least one displayed slice view (12, 22),
   - displaying a volume rendering (17, 31) of the structure of interest (SOI) based on one or more first parameters, said first parameters characterizing the view of the displayed volume rendering (17, 31) of the structure of interest (SOI),

   **characterized in that**
   - said first parameters are determined automatically by considering the user-selected position and one or more second parameters, said second parameters characterizing at least one of the currently displayed slice view (12, 22), wherein the one or more second parameters comprise information about the size of the structure of interest (SOI) in the currently displayed slice view (12, 22), and one or more previous volume renderings of the structure of interest (SOI), and
   - said first parameters are determined **in that** way that an optimized view on the displayed volume rendering (17, 31) of the structure of interest (SOI) is achieved without additional user input apart from the user-selection of the position.

2. Method according to claim 1, wherein the one or more first parameters comprise a viewpoint being a point from which the structure of interest (SOI) is viewed in the displayed volume rendering (17, 31).

3. Method according to claim 1 or 2, wherein the one or more first parameters comprise a viewing direction being a direction in which the structure of interest (SOI) is viewed in the displayed volume rendering (17, 31).

4. Method according to any of the preceding claims, wherein the one or more first parameters comprise at least one clipping surface, each clipping surface separating the structure of interest (SOI) into a first and a second region, wherein details of the structure of interest being in the first region of the clipping surface are displayed in the volume rendering (17, 31) whereas details of the structure of interest (SOI) being in the second region of the clipping surface are not displayed in the volume rendering (17, 31).

5. Method according to claim 3 and 4, wherein at least one clipping surface is a planar clipping plane which is aligned with the viewing direction.

6. Method according to any of the preceding claims, wherein the one or more first parameters comprise a volumetric zoom factor characterizing the size of the structure of interest (SOI) in the displayed volume rendering (17, 31).

7. Method according to any of the preceding claims, wherein the one or more second parameters characterizing an object comprise an orientation (40) of the object, in particular a patient orientation, when the medical data set of the object, in particular of the patient, was acquired.

8. Method according to any of the preceding claims, wherein the one or more second parameters characterizing a structure of interest (SOI) comprise information about the shape of the structure of interest (SOI).

9. Method according to any of the preceding claims, wherein the one or more second parameters characterizing the structure of interest (SOI) comprise information about the visibility of the structure of interest (SOI).

10. Method according to any of the preceding claims, wherein the one or more second parameters characterizing the one or more previous volume renderings (17, 31) of the structure of interest (SOI) comprise information about one or more previous viewpoints being the point or points from which the structure of interest (SOI) was viewed in previously displayed volume rendering or renderings (17, 31) of the structure of interest (SOI).

11. Method according to any of the preceding claims, wherein for at least one of the second parameters an information regarding the quality of viewpoints arising from the at least one of the second parameters is derived.

12. Method according to claim 11, wherein the information regarding the quality of viewpoints is derived by means of calculating a deformed viewing sphere (27, 42, 46, 55, 57, 61, 62) for this second parameter, said deformed viewing sphere (27, 42, 46, 55, 57, 61, 62) corresponding to a sphere surrounding the center of a data set, wherein positions of viewpoints on the surface of the sphere having a higher radial distance are regarded to be better than positions of viewpoints on the surface of the sphere having a lower radial distance.

13. Method according to claim 12, wherein deformed viewing spheres (61, 62) are calculated for two or more second parameters and combined so that a combined deformed viewing sphere (63, 64, 65) is obtained containing information regarding the quality of viewpoints resulting from said two or more second parameters.

14. Method according to claim 13, wherein the deformed viewing spheres (61, 62) for the second parameters are weighted before they are combined to the combined deformed viewing sphere (63, 64, 65).

15. Method according to claim 13 or 14, wherein the deformed viewing spheres (61, 62) are combined by summation, multiplication or thresholding.

16. Method according to any of claims 12 to 15, wherein a good viewpoint is determined by choosing a viewpoint having a position on the surface of the deformed viewing sphere (27, 42, 46, 55, 57, 61, 62) or of the combined deformed viewing sphere (63, 64, 65), respectively, having substantially the highest radial distance.

17. Method according to claims 3 and 16, wherein a good viewing direction is defined by the user-selected position and the good viewpoint.

18. Method according to any claim 12 to 17, wherein a good clipping surface is positioned by considering the deformed viewing sphere (27, 42, 46, 55, 57, 61, 62) or the combined deformed viewing sphere (63, 64, 65), respectively, and by considering an accumulated opacity of the structure of interest (SOI) for rays starting from the user-selected position, wherein the good clipping surface is positioned at a location for which the accumulated opacity is below a given threshold.

19. Method according to claim 6, wherein the volumetric zoom factor is determined considering the size, in particular the slice view zoom factor, of the structure of interest (SOI) in the currently displayed slice view (12, 22).

20. Method according to any of the preceding claims, wherein several positions are user-selected by successively pointing to different positions on the structure of interest (SOI) in the displayed slice view (12, 22) and wherein for each position of the several positions said first parameters are determined automatically and the display of the corresponding volume rendering (17, 31) of the structure of interest (SOI) is updated successively in that way that an optimized view on the displayed volume renderings (17, 31) of the structure of interest (SOI) is achieved without additional user input apart from successively pointing to the different positions on the structure of interest (SOI).

21. Method according to any of the preceding claims, wherein a plurality of positions on the structure of interest (SOI) are user-selected by continuously tracing along the structure of interest (SOI) in the displayed slice view (12, 22) and wherein for each position of the plurality of positions said first parameters are determined automatically and the display of the corresponding volume rendering (17, 31) of the structure of interest (SOI) is updated continuously in that way that an optimized view on the displayed volume renderings (17, 31) of the structure of interest (SOI) is achieved without additional user input apart from continuously tracing along the structure of interest (SOI).

22. Method according to any of the preceding claims, wherein a plurality of positions on the structure of interest (SOI) are selected automatically.

23. Method according to claim 22, wherein the structure of interest (SOI) and its shape and/or run are identified automatically and the positions on the structure of interest (SOI) are positioned automatically along the identified shape and/or run of the structure of interest (SOI).

24. Method according to any of the preceding claims, wherein the automatic determination of said first parameters and the subsequently updated display of the corresponding volume renderings (17, 31) can be activated and deactivated by the user.

25. Method according to claim 24, wherein activation and deactivation happens by pressing and releasing an activation key, in particular a function key or control key, or by selecting an icon on a display (11).

26. Method according to claim 25, wherein the automatic determination of said first parameters and the subsequently updated display of respective volume renderings (17, 31) take place only when the position on the structure of interest (SOI) in the displayed slice view (12, 22) is selected by the user while the activation key is simultaneously pressed by the user.

27. Method according to any of the claims 24 to 26, wherein the user deactivates the automatic determination of said first parameters and the subsequent display of respective volume renderings (17, 31) and amends at least one of the automatically determined first parameters, whereupon an updated volume rendering of the structure of interest is displayed based on the amended first parameters.

28. Method according to any of the claims 12 to 15, wherein at least two deformed viewing spheres (27, 42, 46, 55, 57, 61-65) are obtained from previously acquired medical data sets of one or more objects, in particular one or more patients, both said previously acquired medical data sets and a currently acquired medical data set originating from the same type of examination, and wherein one or more first parameters characterizing the view of the displayed volume rendering (17, 31) of a structure of interest (SOI) of the currently acquired medical data set are determined by considering the at least two deformed viewing spheres (27, 42, 46, 55, 57, 61-65) obtained from the previously acquired medical data sets.

29. Method according to claim 28, wherein said at least two deformed viewing spheres (27, 42, 46, 55, 57, 61-65) obtained from the previously acquired medical data sets are superimposed so that an accumulated deformed viewing sphere is obtained, the first parameters being derived from said accumulated deformed viewing sphere.

30. Method according to claim 28 or 29, wherein at least one of said at least two deformed viewing spheres (27, 42, 46, 55, 57, 61-65) obtained from the previously acquired medical data sets is a combined deformed viewing sphere (63, 64, 65) that is obtained by combining two or more viewing spheres for two or more second parameters, said second parameters characterizing at least one of the previously examined patient, the previously selected structure of interest (SOI), the previously displayed slice and previous volume renderings of the previously selected structure of interest (SOI).

31. Apparatus for volume rendering of medical data sets comprising:

    - a display (11) for displaying at least one slice view (12, 22) of a medical data set of an object, in particular a patient,
    - a user selection unit (13, 14, 15) enabling a user to select a position on a structure of interest (SOI) in the at least one displayed slice view (12, 22), and
    - a volume rendering unit (16) for determining a volume rendering (17, 31) of the structure of interest (SOI) based on one or more first parameters, said first parameters characterizing the view of the displayed volume rendering (17, 31) of the structure of interest (SOI), said volume rendering (17, 31) of the structure of interest (SOI) being displayed on the display (11),
    **characterized in that**
    - said volume rendering unit (16) is designed for determining said first parameters automatically by considering the user-selected position and one or more second parameters, said second parameters characterizing at least one of the currently displayed slice view (12, 22), wherein the one or more second parameters comprise information about the size of the structure of interest (SOI) in the currently displayed slice view (12, 22), and one or more previous volume renderings (17, 31) of the structure of interest (SOI), and

- said volume rendering unit (16) is designed for determining said first parameters **in that** way that an optimized view on the displayed volume rendering (17, 31) of the structure of interest (SOI) is achieved without additional user input apart from the user-selection of the position.

**Patentansprüche**

1. Verfahren zur Volumendarstellung (Volume Rendering) medizinischer Datensätze, das die folgenden Schritte umfasst:

   - Beschaffen eines medizinischen Datensatzes von einem Objekt, insbesondere einem Patienten,
   - Anzeigen zumindest einer Schnittdarstellung (12, 22) des beschafften medizinischen Datensatzes,
   - Auswählen, durch den Benutzer, einer Position auf einer Struktur von Interesse (SOI: Structure of Interest) in der zumindest einen angezeigten Schnittdarstellung (12, 22),
   - Anzeigen einer Volumendarstellung (17, 31) der Struktur von Interesse (SOI) auf Grundlage eines oder mehrerer erster Parameter, wobei die ersten Parameter die Ansicht der angezeigten Volumendarstellung (17, 31) der Struktur von Interesse (SOI) charakterisieren,
   **dadurch gekennzeichnet, dass**
   - die ersten Parameter automatisch bestimmt werden durch Berücksichtigen der vom Benutzer ausgewählten Position und eines oder mehrerer zweiter Parameter, wobei die zweiten Parameter zumindest eines der Folgenden charakterisieren, nämlich die gegenwärtig angezeigte Schnittdarstellung (12, 22), wobei der eine oder die mehreren zweiten Parameter Informationen über die Größe der Struktur von Interesse (SOI) in der gegenwärtig angezeigten Schnittdarstellung (12, 22) umfassen, und eine oder mehrere vorhergehende Volumendarstellungen der Struktur von Interesse (SOI), und
   - die ersten Parameter in solcher Weise bestimmt werden, dass eine optimierte Sicht auf die angezeigte Volumendarstellung (17, 31) der Struktur von Interesse (SOI) erzielt wird ohne zusätzliche Benutzereingabe, ausgenommen die Auswahl der Position durch den Benutzer.

2. Verfahren nach Anspruch 1, wobei der eine oder die mehreren ersten Parameter einen Blickpunkt umfassen, der ein Punkt ist, von dem aus die Struktur von Interesse (SOI) in der angezeigten Volumendarstellung (17, 31) betrachtet wird.

3. Verfahren nach Anspruch 1 oder 2, wobei der eine oder die mehreren Parameter eine Blickrichtung umfassen, die eine Richtung ist, in der die Struktur von Interesse (SOI) in der angezeigten Volumendarstellung (17, 31) betrachtet wird.

4. Verfahren nach einem der vorstehenden Ansprüche, wobei der eine oder die mehreren ersten Parameter zumindest eine Clippingfläche umfassen, wobei jede Clippingfläche die Struktur von Interesse (SOI) in einen ersten und einen zweiten Bereich trennt, wobei Einzelheiten der Struktur von Interesse, die sich im ersten Bereich der Clippingfläche befinden, in der Volumendarstellung (17, 31) angezeigt werden, wohingegen Einzelheiten der Struktur von Interesse (SOI), die sich im zweiten Bereich der Clippingfläche befinden, nicht in der Volumendarstellung (17, 31) angezeigt werden.

5. Verfahren nach Anspruch 3 und 4, wobei zumindest eine Clippingfläche eine plane Clippingebene ist, die an der Blickrichtung ausgerichtet ist.

6. Verfahren nach einem der vorstehenden Ansprüche, wobei der eine oder die mehreren ersten Parameter einen volumetrischen Zoomfaktor umfassen, der die Größe der Struktur von Interesse (SOI) in der angezeigten Volumendarstellung (17, 31) charakterisiert.

7. Verfahren nach einem der vorstehenden Ansprüche, wobei der eine oder die mehreren zweiten Parameter, die ein Objekt charakterisieren, eine Ausrichtung (40) des Objekts, insbesondere eine Patientenausrichtung, als der medizinische Datensatz des Objekts, insbesondere des Patienten, beschafft wurde, umfassen.

8. Verfahren nach einem der vorstehenden Ansprüche, wobei der eine oder die mehreren zweiten Parameter, die eine Struktur von Interesse (SOI) charakterisieren, Informationen über die Form der Struktur von Interesse (SOI) umfassen.

9. Verfahren nach einem der vorstehenden Ansprüche, wobei der eine oder die mehreren zweiten Parameter, welche die Struktur von Interesse (SOI) charakterisieren, Informationen über die Sichtbarkeit der Struktur von Interesse (SOI) umfassen.

10. Verfahren nach einem der vorstehenden Ansprüche, wobei der eine oder die mehreren zweiten Parameter, welche die eine oder mehreren vorhergehenden Volumendarstellungen (17, 31) der Struktur von Interesse (SOI) charakterisieren, Informationen über einen oder mehrere Blickpunkte umfassen, also den Punkt oder die Punkte, von dem bzw. denen aus die Struktur von Interesse (SOI) betrachtet wurde in einer zuvor angezeigten Volumendarstellung oder -darstellungen (17, 31) der Struktur von Interesse (SOI).

11. Verfahren nach einem der vorstehenden Ansprüche, wobei für zumindest einen der zweiten Parameter eine Information bezüglich der Qualität von Blickpunkten, die sich aus dem zumindest einen der zweiten Parameter ergibt, abgeleitet wird.

12. Verfahren nach Anspruch 11, wobei die Informationen bezüglich der Qualität von Blickpunkten abgeleitet werden durch Berechnen einer verformten Sichtkugel (27, 42, 46, 55, 57, 61, 62) für diesen zweiten Parameter, wobei die verformte Sichtkugel (27, 42, 46, 55, 57, 61, 62) einer Kugel entspricht, welche die Mitte eines Datensatzes umgibt, wobei Positionen von Blickpunkten auf der Oberfläche der Kugel, die einen größeren radialen Abstand aufweisen, besser eingestuft werden als Positionen von Blickpunkten auf der Oberfläche der Kugel, die einen geringeren radialen Abstand aufweisen.

13. Verfahren nach Anspruch 12, wobei verformte Sichtkugeln (61, 62) für zwei oder mehr zweite Parameter berechnet und kombiniert werden, so dass eine kombinierte verformte Sichtkugel (63, 64, 65) erhalten wird, welche Informationen bezüglich der Qualität von Blickpunkten enthält, die sich aus den zwei oder mehr zweiten Parametern ergibt.

14. Verfahren nach Anspruch 13, wobei die verformten Sichtkugeln (61, 62) für die zweiten Parameter gewichtet werden, bevor sie zur kombinierten verformten Sichtkugel (63, 64, 65) kombiniert werden.

15. Verfahren nach Anspruch 13 oder 14, wobei die verformten Sichtkugeln (61, 62) durch Summenbildung, Multiplikation oder Schwellenwertbildung kombiniert werden.

16. Verfahren nach einem der Ansprüche 12 bis 15, wobei ein guter Blickpunkt bestimmt wird durch Auswählen eines Blickpunkts mit jeweils einer Position auf der Oberfläche der verformten Sichtkugel (27, 42, 46, 55, 57, 61, 62) oder der kombinierten verformten Sichtkugel (63, 64, 65), der im Wesentlichen den größten radialen Abstand aufweist.

17. Verfahren nach Anspruch 3 und 16, wobei eine gute Blickrichtung durch die vom Benutzer ausgewählte Position und den guten Blickpunkt festgelegt wird.

18. Verfahren nach einem der Ansprüche 12 bis 17, wobei eine gute Clippingfläche positioniert wird durch jeweiliges Berücksichtigen der verformten Sichtkugel (27, 42, 46, 55, 57, 61, 62) oder der kombinierten verformten Sichtkugel (63, 64, 65) und durch Berücksichtigen einer akkumulierten Opazität der Struktur von Interesse (SOI) für Strahlen ab der vom Benutzer ausgewählten Position, wobei die gute Clippingfläche an einem Ort positioniert ist, für den die akkumulierte Opazität unter einem gegebenen Schwellenwert liegt.

19. Verfahren nach Anspruch 6, wobei der volumetrische Zoomfaktor bestimmt wird unter Berücksichtigung der Größe, insbesondere des Schnittdarstellungs-Zoomfaktors, der Struktur von Interesse (SOI) in der gegenwärtig angezeigten Schnittdarstellung (12, 22).

20. Verfahren nach einem der vorstehenden Ansprüche, wobei mehrere Positionen vom Benutzer ausgewählt werden durch sukzessives Zeigen auf verschiedene Positionen auf der Struktur von Interesse (SOI) in der angezeigten Schnittdarstellung (12, 22) und wobei für jede Position aus den mehreren Positionen die ersten Parameter automatisch bestimmt werden und die Anzeige der entsprechenden Volumendarstellung (17, 31) der Struktur von Interesse (SOI) sukzessive aktualisiert wird in solcher Weise, dass eine optimierte Sicht auf die angezeigten Volumendarstellungen (17, 31) der Struktur von Interesse (SOI) erzielt wird ohne zusätzliche Benutzereingabe, ausgenommen das sukzessive Zeigen auf die verschiedenen Positionen auf der Struktur von Interesse (SOI).

21. Verfahren nach einem der vorstehenden Ansprüche, wobei eine Mehrzahl von Positionen auf der Struktur von Interesse (SOI) vom Benutzer ausgewählt wird durch kontinuierliches Entlangfahren an der Struktur von Interesse (SOI) in der angezeigten Schnittdarstellung (12, 22) und wobei für jede Position aus der Mehrzahl von Positionen die ersten Parameter automatisch bestimmt werden und die Anzeige der entsprechenden Volumendarstellung (17, 31) der Struktur von Interesse (SOI) fortlaufend aktualisiert wird in solcher Weise, dass eine optimierte Sicht auf die angezeigten Volumendarstellungen (17, 31) der Struktur von Interesse (SOI) erzielt wird ohne zusätzliche Benutzereingabe, ausgenommen das kontinuierliche Entlangfahren an der Struktur von Interesse (SOI).

22. Verfahren nach einem der vorstehenden Ansprüche, wobei eine Mehrzahl von Positionen auf der Struktur von Interesse (SOI) automatisch ausgewählt wird.

23. Verfahren nach Anspruch 22, wobei die Struktur von Interesse (SOI) und ihre Form und/oder ihr Verlauf automatisch identifiziert werden und die Positionen auf der Struktur von Interesse (SOI) automatisch entlang der identifizierten Form und/oder dem identifizierten Verlauf der Struktur von Interesse (SOI) angeordnet werden.

24. Verfahren nach einem der vorstehenden Ansprüche, wobei die automatische Bestimmung der ersten Parameter und die anschließende aktualisierte Anzeige der entsprechenden Volumendarstellungen (17, 31) vom Benutzer aktiviert und deaktiviert werden können.

25. Verfahren nach Anspruch 24, wobei Aktivierung und Deaktivierung erfolgen durch Drücken und Freigeben einer Aktivierungstaste, insbesondere einer Funktionstaste oder Steuertaste, oder durch Auswählen eines Sinnbildes auf einer Anzeige (11).

26. Verfahren nach Anspruch 25, wobei die automatische Bestimmung der ersten Parameter und die anschließende aktualisierte Anzeige jeweiliger Volumendarstellungen (17, 31) nur stattfinden, wenn die Position auf der Struktur von Interesse (SOI) in der angezeigten Schnittdarstellung (12, 22) vom Benutzer ausgewählt wird, während die Aktivierungstaste gleichzeitig vom Benutzer gedrückt wird.

27. Verfahren nach einem der Ansprüche 24 bis 26, wobei der Benutzer die automatische Bestimmung der ersten Parameter und die anschließende Anzeige jeweiliger Volumendarstellungen (17, 31) deaktiviert und zumindest einen der automatisch bestimmten ersten Parameter ändert, woraufhin eine aktualisierte Volumendarstellung der Struktur von Interesse angezeigt wird auf Grundlage der geänderten ersten Parameter.

28. Verfahren nach einem der Ansprüche 12 bis 15, wobei zumindest zwei verformte Sichtkugeln (27, 42, 46, 55, 57, 61-65) erhalten werden aus zuvor beschafften medizinischen Datensätzen von einem oder mehreren Objekten, insbesondere einem oder mehreren Patienten, wobei sowohl die zuvor beschafften medizinischen Datensätze als auch ein gegenwärtig beschaffter medizinischer Datensatz vom gleichen Typ Untersuchung herrühren, und wobei einer oder mehrere erste Parameter, welche die Ansicht der angezeigten Volumendarstellung (17, 31) einer Struktur von Interesse (SOI) des gegenwärtig beschafften medizinischen Datensatzes charakterisieren, bestimmt werden durch Berücksichtigen der zumindest zwei verformten Sichtkugeln (27, 42, 46, 55, 57, 61-65), erhalten aus den zuvor beschafften medizinischen Datensätzen.

29. Verfahren nach Anspruch 28, wobei die zumindest zwei verformten Sichtkugeln (27, 42, 46, 55, 57, 61-65), erhalten aus den zuvor beschafften medizinischen Datensätzen, übereinandergelagert werden, so dass eine akkumulierte verformte Sichtkugel erhalten wird, wobei die ersten Parameter von der akkumulierten verformten Sichtkugel abgeleitet werden.

30. Verfahren nach Anspruch 28 oder 29, wobei zumindest eine der zumindest zwei verformten Sichtkugeln (27, 42, 46, 55, 57, 61-65), erhalten aus den zuvor beschafften medizinischen Datensätzen, eine kombinierte verformte Sichtkugel (63, 64, 65) ist, die erhalten wird durch Kombinieren zweier oder mehr Sichtkugeln für zwei oder mehr zweite Parameter, wobei die zweiten Parameter zumindest eines charakterisieren von dem zuvor untersuchten Patienten, der zuvor ausgewählten Struktur von Interesse (SOI), der zuvor angezeigten Schnittdarstellung und vorhergehenden Volumendarstellungen der zuvor ausgewählten Struktur von Interesse (SOI).

31. Vorrichtung zur Volumendarstellung (Volume Rendering) medizinischer Datensätze, umfassend:

- eine Anzeige (11) zum Anzeigen zumindest einer Schnittdarstellung (12, 22) eines medizinischen Datensatzes von einem Objekt, insbesondere einem Patienten,
- eine Benutzerauswahleinheit (13, 14, 15), die einen Benutzer befähigt, eine Position auf einer Struktur von Interesse (SOI: Structure of Interest) in der zumindest einen angezeigten Schnittdarstellung (12, 22) auszuwählen, und

- eine Volumendarstellungseinheit (16) zur Bestimmung einer Volumendarstellung (17, 31) der Struktur von Interesse (SOI) auf Grundlage eines oder mehrerer erster Parameter, wobei die ersten Parameter die Ansicht der angezeigten Volumendarstellung (17, 31) der Struktur von Interesse (SOI) charakterisieren, wobei die Volumendarstellung (17, 31) der Struktur von Interesse (SOI) auf der Anzeige (11) angezeigt wird,
**dadurch gekennzeichnet, dass**
- die Volumendarstellungseinheit (16) entworfen ist zur automatischen Bestimmung der ersten Parameter durch Berücksichtigen der vom Benutzer ausgewählten Position und eines oder mehrerer zweiter Parameter, wobei die zweiten Parameter zumindest eines der Folgenden charakterisieren, nämlich die gegenwärtig angezeigte Schnittdarstellung (12, 22), wobei der eine oder die mehreren zweiten Parameter Informationen über die Größe der Struktur von Interesse (SOI) in der gegenwärtig angezeigten Schnittdarstellung (12, 22) umfassen, und eine oder mehrere vorhergehende Volumendarstellungen (17, 31) der Struktur von Interesse (SOI), und
- die Volumendarstellungseinheit (16) entworfen ist zur Bestimmung der ersten Parameter in solcher Weise, dass eine optimierte Sicht auf die angezeigte Volumendarstellung (17, 31) der Struktur von Interesse (SOI) erzielt wird ohne zusätzliche Benutzereingabe, ausgenommen die Auswahl der Position durch den Benutzer.

## Revendications

1.  Procédé de rendu de volume d'ensembles de données médicales comprenant les étapes suivantes :

    - l'acquisition d'un ensemble de données médicales d'un objet, notamment d'un patient,
    - l'affichage d'au moins une vue en coupe (12, 22) de l'ensemble de données médicales acquis,
    - la sélection par un utilisateur d'une position existant sur une structure d'intérêt (SOI) dans l'au moins une vue en coupe affichée (12, 22),
    - l'affichage d'un rendu de volume (17, 31) de la structure d'intérêt (SOI) fondé sur un ou plusieurs premiers paramètres, lesdits premiers paramètres caractérisant la vue du rendu de volume affiché (17, 31) de la structure d'intérêt (SOI),
    **caractérisé en ce que**
    - lesdits premiers paramètres sont déterminés de manière automatique en prenant en considération la position sélectionnée par l'utilisateur et un ou plusieurs seconds paramètres, lesdits seconds paramètres caractérisant la vue en coupe actuellement affichée (12, 22), les un ou plusieurs seconds paramètres comprenant une information concernant la taille de la structure d'intérêt (SOI) dans la vue en coupe actuellement affichée (12, 22), et/ou caractérisant un ou plusieurs rendus de volume précédents de la structure d'intérêt (SOI), et
    - lesdits premiers paramètres sont déterminés de manière qu'une vue optimisée sur le rendu de volume affiché (17, 31) de la structure d'intérêt (SOI) soit possible sans autre intervention de la part de l'utilisateur que la sélection de la position.

2.  Procédé selon la revendication 1, dans lequel les un ou plusieurs premiers paramètres comprennent un point de vue consistant en un point à partir duquel la structure d'intérêt (SOI) est vue dans le rendu de volume affiché (17, 31).

3.  Procédé selon la revendication 1 ou 2, dans lequel les un ou plusieurs premiers paramètres comprennent un sens de visualisation consistant en un sens dans lequel la structure d'intérêt (SOI) est vue dans le rendu de volume affiché (17, 31).

4.  Procédé selon l'une quelconque des revendications précédentes, dans lequel les un ou plusieurs premiers paramètres comprennent au moins une surface de découpage, chaque surface de découpage séparant la structure d'intérêt (SOI) en une première et une seconde région, dans lequel les détails de la structure d'intérêt se trouvant dans la première région de la surface de découpage sont affichés dans le rendu de volume (17, 31) tandis que les détails de la structure d'intérêt (SOI) se trouvant dans la seconde région de la surface de découpage ne sont pas affichés dans le rendu de volume (17, 31).

5.  Procédé selon les revendications 3 et 4, dans lequel au moins une surface de découpage consiste en un plan de découpage plan aligné avec le sens de visualisation.

6.  Procédé selon l'une quelconque des revendications précédentes, dans lequel les un ou plusieurs premiers paramètres comprennent un facteur de magnification volumétrique caractérisant la taille de la structure d'intérêt (SOI) dans le rendu de volume affiché (17, 31).

7.  Procédé selon l'une quelconque des revendications précédentes, dans lequel les un ou plusieurs seconds paramètres caractérisant un objet comprennent une orientation (40) de l'objet, notamment l'orientation d'un patient, au moment de l'acquisition de l'ensemble de données médicales de l'objet, notamment du patient.

8.  Procédé selon l'une quelconque des revendications précédentes, dans lequel les un ou plusieurs seconds paramètres caractérisant une structure d'intérêt (SOI) comprennent une information concernant la forme de la structure d'intérêt (SOI).

9.  Procédé selon l'une quelconque des revendications précédentes, dans lequel les un ou plusieurs seconds paramètres caractérisant la structure d'intérêt (SOI) comprennent une information concernant la visibilité de la structure d'intérêt (SOI).

10. Procédé selon l'une quelconque des revendications précédentes, dans lequel les un ou plusieurs seconds paramètres caractérisant les un ou plusieurs rendus de volume précédents (17, 31) de la structure d'intérêt (SOI) comprennent une information concernant un ou plusieurs points de vue précédents consistant en un ou des points à partir desquels la structure d'intérêt (SOI) a été vue dans le ou les rendu(s) de volume précédemment affiché(s) (17, 31) de la structure d'intérêt (SOI).

11. Procédé selon l'une quelconque des revendications précédentes, dans lequel on dérive, pour au moins un des seconds paramètres, une information concernant la qualité des points de vue résultant de l'au moins un des seconds paramètres.

12. Procédé selon la revendication 11, dans lequel l'information concernant la qualité des points de vue est dérivée par calcul d'une sphère de visualisation déformée (27, 42, 46, 55, 57, 61, 62) pour ce second paramètre, ladite sphère de visualisation déformée (27, 42, 46, 55, 57, 61, 62) correspondant à une sphère entourant le centre d'un ensemble de données, dans lequel les positions des points de vue dirigés sur la surface de la sphère ayant une plus grande distance radiale sont considérées meilleures que les positions des points de vue dirigés sur la surface de la sphère ayant une plus faible distance radiale.

13. Procédé selon la revendication 12, dans lequel des sphères de visualisation déformées (61, 62) sont calculées pour deux ou plus de deux seconds paramètres et sont combinées de manière à produire une sphère de visualisation déformée combinée (63, 64, 65) contenant une information concernant la qualité des points de vue résultant desdits deux ou plus de deux seconds paramètres.

14. Procédé selon la revendication 13, dans lequel les sphères de visualisation déformées (61, 62) pour les seconds paramètres sont pondérées avant d'être combinées pour former la sphère de visualisation déformée combinée (63, 64, 65).

15. Procédé selon la revendication 13 ou 14, dans lequel les sphères de visualisation déformées (61, 62) sont combinées par sommation, multiplication ou seuillage.

16. Procédé selon l'une quelconque des revendications 12 à 15, dans lequel un point de vue avantageux est déterminé en choisissant un point de vue dont la position sur la surface de la sphère de visualisation déformée (27, 42, 46, 55, 57, 61, 62) ou de la sphère de visualisation déformée combinée (63, 64, 65), respectivement, a la distance radiale sensiblement la plus grande.

17. Procédé selon les revendications 3 et 16, dans lequel un sens de visualisation avantageux est défini par la position sélectionnée par l'utilisateur et le point de vue avantageux.

18. Procédé selon l'une quelconque des revendications 12 à 17, dans lequel une surface de découpage avantageuse est positionnée en considérant la sphère de visualisation déformée (27, 42, 46, 55, 57, 61, 62) ou la sphère de visualisation déformée combinée (63, 64, 65), respectivement, et en considérant une opacité accumulée de la structure d'intérêt (SOI) pour des rayons partant de la position sélectionnée par l'utilisateur, la surface de découpage avantageuse étant positionnée en un emplacement auquel l'opacité accumulée est inférieure à un seuil donné.

19. Procédé selon la revendication 6, dans lequel le facteur de magnification volumétrique est déterminé en considérant la taille, notamment le facteur de magnification de la vue en coupe, de la structure d'intérêt (SOI) dans la vue en coupe actuellement affichée (12, 22).

**20.** Procédé selon l'une quelconque des revendications précédentes, dans lequel plusieurs positions sont sélectionnées par l'utilisateur en pointant successivement sur différentes positions sur la structure d'intérêt (SOI) dans la vue en coupe affichée (12, 22) et dans lequel, pour chaque position parmi les plusieurs positions, lesdits premiers paramètres sont déterminés de manière automatique et l'affichage du rendu de volume correspondant (17, 31) de la structure d'intérêt (SOI) est successivement réactualisé de manière à produire une vue optimisée sur les rendus de volume affichés (17, 31) de la structure d'intérêt (SOI) sans autre intervention de la part de l'utilisateur que le pointage successif sur les différentes positions existant sur la structure d'intérêt (SOI).

**21.** Procédé selon l'une quelconque des revendications précédentes, dans lequel une pluralité de positions existant sur la structure d'intérêt (SOI) sont sélectionnées par l'utilisateur en effectuant un tracé continu le long de la structure d'intérêt (SOI) dans la vue en coupe affichée (12, 22) et dans lequel, pour chaque position parmi la pluralité de positions, lesdits premiers paramètres sont déterminés de manière automatique et l'affichage du rendu de volume correspondant (17, 31) de la structure d'intérêt (SOI) est réactualisé en continu de manière à produire une vue optimisée sur les rendus de volume affichés (17, 31) de la structure d'intérêt (SOI) sans autre intervention de la part de l'utilisateur que le tracé continu le long de la structure d'intérêt (SOI).

**22.** Procédé selon l'une quelconque des revendications précédentes, dans lequel une pluralité de positions existant sur la structure d'intérêt (SOI) sont sélectionnées de manière automatique.

**23.** Procédé selon la revendication 22, dans lequel la structure d'intérêt (SOI) et sa forme et/ou sa course sont identifiées automatiquement et les positions existant sur la structure d'intérêt (SOI) sont positionnées automatiquement le long de la forme et/ou de la course identifiées de la structure d'intérêt (SOI).

**24.** Procédé selon l'une quelconque des revendications précédentes, dans lequel la détermination automatique desdits premiers paramètres et l'affichage subséquemment réactualisé des rendus de volume correspondants (17, 31) peuvent être activés et désactivés par l'utilisateur.

**25.** Procédé selon la revendication 24, dans lequel l'activation et la désactivation se produisent en appuyant et en relâchant une touche d'activation, notamment une touche de fonction ou une touche de commande, ou en sélectionnant une icône sur un dispositif d'affichage (11).

**26.** Procédé selon la revendication 25, dans lequel la détermination automatique desdits premiers paramètres et l'affichage subséquemment réactualisé des rendus de volume respectifs (17, 31) a lieu uniquement lorsque la position existant sur la structure d'intérêt (SOI) dans la vue en coupe affichée (12, 22) est sélectionnée par l'utilisateur tandis qu'il appuie simultanément sur la touche d'activation.

**27.** Procédé selon l'une quelconque des revendications 24 à 26, dans lequel l'utilisateur désactive la détermination automatique desdits premiers paramètres et l'affichage subséquent des rendus de volume respectifs (17, 31) et modifie au moins l'un des premiers paramètres automatiquement déterminés, donnant lieu à un affichage du rendu de volume de la structure d'intérêt modifié, fondé sur les premiers paramètres modifiés.

**28.** Procédé selon l'une quelconque des revendications 12 à 15, dans lequel au moins deux sphères de visualisation déformées (27, 42, 46, 55, 57, 61-65) sont produites à partir d'ensembles de données médicales précédemment acquis relatifs à un ou plusieurs objets, notamment à un ou plusieurs patients, lesdits deux ensembles de données médicales précédemment acquis et un ensemble de données médicales actuellement acquis provenant du même type d'examen, et dans lequel un ou plusieurs premiers paramètres caractérisant la vue du rendu de volume affiché (17, 31) d'une structure d'intérêt (SOI) de l'ensemble de données médicales actuellement acquis sont déterminés en considérant les au moins deux sphères de visualisation déformées (27, 42, 46, 55, 57, 61-65) produites à partir des ensembles de données médicales précédemment acquis.

**29.** Procédé selon la revendication 28, dans lequel lesdites au moins deux sphères de visualisation déformées (27, 42, 46, 55, 57, 61-65) produites à partir des ensembles de données médicales précédemment acquis sont superposées de manière à produire une sphère de visualisation déformée accumulée, les premiers paramètres étant dérivés de ladite sphère de visualisation déformée accumulée.

**30.** Procédé selon la revendication 28 ou 29, dans lequel au moins une desdites au moins deux sphères de visualisation déformées (27, 42, 46, 55, 57, 61-65) produites à partir des ensembles de données médicales précédemment acquis consiste en une sphère de visualisation déformée combinée (63, 64, 65) obtenue en combinant deux ou plus de deux sphères de visualisation pour deux ou plus de deux seconds paramètres, lesdits seconds paramètres caractérisant le patient précédemment examiné et/ou la structure d'intérêt (SOI) précédemment sélectionnée et/ou la coupe précédemment affichée et/ou les précédents rendus de volume de la structure d'intérêt (SOI) précédemment sélectionnée.

31. Appareil de rendu de volume d'ensembles de données médicales comprenant :

- un dispositif d'affichage (11) destiné à afficher au moins une vue en coupe (12, 22) d'un ensemble de données médicales d'un objet, notamment d'un patient,
- une unité de sélection utilisateur (13, 14, 15) permettant à un utilisateur de sélectionner une position existant sur une structure d'intérêt (SOI) dans l'au moins une vue en coupe affichée (12, 22), et
- une unité de rendu de volume (16) servant à déterminer un rendu de volume (17, 31) de la structure d'intérêt (SOI) en se fondant sur un ou plusieurs premiers paramètres, lesdits premiers paramètres caractérisant la vue du rendu de volume affiché (17, 31) de la structure d'intérêt (SOI), ledit rendu de volume (17, 31) de la structure d'intérêt (SOI) étant affiché sur le dispositif d'affichage (11),
**caractérisé en ce que**
- ladite unité de rendu de volume (16) est conçue pour déterminer lesdits premiers paramètres de manière automatique en considérant la position sélectionnée par l'utilisateur et un ou plusieurs seconds paramètres, lesdits seconds paramètres caractérisant la vue en coupe actuellement affichée (12, 22), les un ou plusieurs seconds paramètres comprenant une information concernant la taille de la structure d'intérêt (SOI) dans la vue en coupe actuellement affichée (12, 22), et/ou caractérisant un ou plusieurs rendus de volume précédents (17, 31) de la structure d'intérêt (SOI), et
- ladite unité de rendu de volume (16) est conçue pour déterminer lesdits premiers paramètres de manière qu'une vue optimisée sur le rendu de volume affiché (17, 31) de la structure d'intérêt (SOI) soit possible sans autre intervention de la part de l'utilisateur que la sélection de la position.

Fig. 1

Fig. 2

Fig. 3

Fig. 4

Fig. 5

Fig. 6

Fig. 7

Fig. 8

Fig. 9

Fig. 10

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Virtual Terrain Project.,* March 2007, http://www.vterrain.org/Textures/spherical.html
- **V. Blanz ; M. J. Tarr ; H. H. Bülthoff.** What object attributes determine canonical views?. *Perception,* 1999, vol. 28, 575-599
- **U. D. Bordoloi ; H.-W. Shen.** View selection for volume rendering. *IEEE Visualization '05,* 2005, 487-494
- **P. Bourke.** *Distributing Points on a Sphere.,* March 2007
- **M.-Y. Chan ; H. Qu ; Y. Wu ; H. Zhou.** Viewpoint selection for angiographic. *International Symposium on Visual Computing '06,* 2006, 528-537
- **S. Fleishman ; D. Cohen-Or ; D. Lischinski.** Automatic camera placement for image-based modeling. *Computer Graphics Forum,* 2000, vol. 19 (2), 101-110
- **C. H. Lee ; A. Varshney ; D. W. Jacobs.** Mesh saliency. *ACM SIGGRAPH '05,* 2005, 659-666
- **K. Mühler ; M. Neugebauer ; C. Tietjen ; B. Preim.** Viewpoint selection for intervention planning. *Eurographics/IEEE VGTC Symposium on Visualization,* 2007, 267-274
- **S. Owada ; F. Nielsen ; T. Igarashi.** *Symposium on Interactive 3D Graphics and Games '05,* 2005, 111-116
- **O. Polonsky ; G. Patané ; S. Biasotti ; C. Gotsman ; M. Spagnuolo.** What's in an image: Towards the computation of the "best" view of an object. *The Visual Computer,* 2005, vol. 21 (8-10), 840-847
- **Y. Sato ; C.-F. Westin ; A. Bhalerao ; S. Nakajima ; N. Shiraga ; S. Tamura ; R. Kikinis.** Tissue classification based on 3D local intensity structures for volume rendering. *IEEE Transactions on Visualization and Computer Graphics,* 2000, vol. 6 (2), 160-180
- **M. Sbert ; D. Plemenos ; M. Feixas ; F. González.** Viewpoint quality: Measures and applications. *Computational Aesthetics '05,* 2005, 185-192
- **S. Takahashi ; I. Fujishiro ; Y. Takeshima ; T. Nishita.** A feature-driven approach to locating optimal viewpoints for volume visualization. *IEEE Visualization '05,* 2005, 495-502
- **M. Tory ; C. Swindells.** Comparing ExoVis, orientation icon, and inplace 3D visualization techniques. *Graphics Interface '03,* 2003, 57-64
- **J. M. van Verth ; L. M. Bishop.** Essential Mathematics for Games and Interactive Applications. Morgan Kaufmann Publishers, 2004
- **P.-P. Vazquez ; M. Feixas ; M. Sbert ; W. Heidrich.** Viewpoint selection using viewpoint entropy. *VMV '01,* 2001, 273-280
- **P.-P. Vázquez ; M. Feixas ; M. Sbert ; W. Heidrich.** Automatic view selection using viewpoint entropy and its application to image-based modelling. *Computer Graphics Forum,* 2003, vol. 22 (4), 689-700
- **I. Viola ; M. Feixas ; M. Sbert ; M. E. Gröller.** Importance-driven focus of attention. *IEEE Transactions on Visualization and Computer Graphics,* 2006, vol. 12 (5), 933-940
- **E. Williams.** *Aviation Formulary,* March 2007, vol. 1.43, hftp://williams.best.vwh.net/avform.html
- **J. Zhou ; M. Hinz ; K. D. Tönnies.** Focal region-guided feature-based volume rendering. *1st International Symposium on 3D Data Processing, Visualization and Transmission '02,* 2002, 87-90